# EUROPEAN PATENT APPLICATION

(11) **EP 3 958 205 A1**
(43) Date of publication of application: **23.02.2022**
(21) Application number: 19925346.9
(22) Date of filing: 17.04.2019
(51) Int. Cl.: G06Q 50/04, G06Q 10/08, G06Q 30/00, G01N 21/25, G01N 21/78, G06K 9/46, G06K 19/06

(54) **FOOD FRESHNESS MONITORING METHOD AND SYSTEM**

(71) Applicant: Greens Systems Co., Ltd., Seoul 08212 (KR)
(72) Inventor: NAM, Seongwon, Seoul 07340 (KR)
(74) Representative: Regimbeau
(86) International application number: PCT/KR2019/004657
(87) International publication number: WO 2020/213761

(57) **Abstract**

The present invention relates to a freshness guide method and a freshness guide system capable of providing guidance with respect to the freshness of food sealed in a packaging container. The freshness guide method and the freshness guide system according to the present invention can accurately provide a user with freshness information regarding food by using loT, RFID, big data, and artificial intelligence (Al) technologies leading the 4th industrial revolution.

## Description

### FIELD

The present disclosure relates to a freshness monitoring method and system capable of monitoring freshness of food.

### BACKGROUND ART

The global food market in 2015 reached about $5.6 trillion, which was larger than the information technology (IT) and automobile markets put together. It is a huge market that is expected to have grown to $6.3 trillion in 2018. The global packaging market, including food packaging, is valued at approximately $670 billion and has grown by 3% annually to $840 billion in 2016. The global packaging market is expected to continue to grow in the future. Among others, a smart food distribution system employing a sensor-based loT (Internet of Thing) technology that detects temperature, humidity, and location information in real time throughout the entire process of food production, transportation, storage, and sales is attracting attention as one of the most important technologies for the next generation.

The smart food distribution system is the next-generation food safety management system that can manage various information related to food, such as history, distribution, nutrition, safety, quality, freshness, and the like to provide to suppliers, consumers, and distributors. It refers to a future safe food distribution system that converges u-IT/BT technologies such as information and communication technology (ICT), biotechnology, sensing technology, and communication technology.

As a technology to be applied to a smart food distribution system, Radio Frequency Identification (RFID), that is, a wireless recognition technology is attracting attention. RFID is a technology that can identify objects (things, people, etc.) using radio frequency (RF). It is a technology of storing information suitable for the purpose of use in an RF tag having an antenna and a chip, attaching the RF tag to an object to be applied, and recognizing information through an RFID reader corresponding to a reader. Recently, RFID has been applied to various fields such as transportation cards, parking management, library management, access control cards, animal identification, high-pass cards, etc.

RFID is considered as a next-generation recognition technology to replace barcodes which is used for product management in the distribution field. Specifically, information regarding the entire process of production, distribution, storage, and consumption is stored in a tag attached to a product, and is read by a reader through an antenna.

Unlike barcodes, RFID can track an entire process until products are released in a finished state from a factory and displayed on shelves of a supermarket. When a consumer selects an item with this tag, the payment for the item is automatically made and even inventory and consumer preference are also comprehensively managed.

However, RFID has not yet been used as a method of managing storage quality of goods. For example, it is impossible to track whether products are being properly managed at an appropriate temperature and humidity or deteriorated while those products are moved from a factory to a warehouse using a vehicle and until the products are displayed on shelves and selected by consumers.

In particular, as refrigerated distribution increases due to increased demands for fresh foods, this problem is becoming more serious in such refrigerated food. In the case of food, a lot of spoiled products are generated due to inappropriate delivery during a distribution process, and returns greatly increase due to these spoiled products, resulting in huge economic losses.

### DISCLOSURE

### TECHNICAL PROBLEM

The present disclosure is directed to solving the aforementioned problems and other drawbacks.

One aspect of the present disclosure is to provide a freshness guide method and a freshness guide system, capable of providing guidance information related to freshness of food sealed in a packaging container by using an Internet of Thing (loT) technology.

Another aspect of the present disclosure is to provide a freshness guide method and a freshness guide system, capable of providing various users with freshness information regarding foods by applying artificial intelligence (Al) to big data accumulatively stored in a server by an loT technology.

### TECHNICAL SOLUTION

According to the present disclosure, there are provided a packaging container for sealing food, and a freshness guide method and system capable of guiding freshness of the food sealed in the packaging container.

First, the freshness monitoring system may include a gas sensor (200) having a food identification code for identifying food sealed in a food accommodation space, and to generate gas information by sensing a gas changed by the food in the food accommodation space, a mobile terminal (100) to search for the food identification code and the gas information using at least one of a camera and a short-range communication module, and to output the food identification code and the gas information through a wireless communication unit, and a server (300) to receive the food identification code and the gas information from the mobile terminal 100 and store the same in a memory. The mobile terminal (100) may output the freshness information related to a target food corresponding to the food identification code based on the gas information in at least one of visual, auditory and tactile manners.

According to one implementation, the gas sensor 200 may change in color due to the gas in the food accommodation space.

According to one implementation, the mobile terminal (100) may search for the food identification code from an image captured by the camera, searches for at least one color code corresponding to the target food from the image, and the freshness information related to the target food may be determined by the color code.

According to one implementation, the mobile terminal (100) may search for a partial region including the food identification code from an entire region of the image, determine a color code extraction region from the entire region of the image based on size and location of the partial region, and search for the color code from the color code extraction region.

According to one implementation, the mobile terminal (100) may search for at least one region having a color corresponding to the target food from the color code extraction region, calculate an average color of the searched at least one region, and determine the calculated average color as the color code. The color may change depending on the target food.

According to one implementation, the mobile terminal (100) may display the image on a display, and output a graphic object on the image to guide the color code extraction region.

According to one implementation, the server (300) may determine a freshness criterion of the target food based on the food identification code, and generate the freshness information related to the target food by applying the color code to the freshness criterion.

According to one implementation, the number of the color code searched for from the image may vary depending on the target food.

According to one implementation, the mobile terminal (100) may apply power to the gas sensor (200), in response to being tagged with the gas sensor (200), and the gas sensor (200) may generate a difference in electrical conductivity through reaction with the gas in the food accommodation space when the power is applied.

According to one implementation, the gas sensor (200) may include a substrate, an antenna and a signal processing module disposed on the substrate, an electrode pair disposed on the substrate and electrically connected to the signal processing module to receive power from the signal processing module, a conductive thin film disposed on the electrode pair, and a gas sensing film disposed to surround the conductive thin film and to generate a difference in electrical conductivity through gas sensing reaction by receiving power from the electrode pair.

Furthermore, the freshness guide method may guide freshness of food using a mobile terminal having a camera and a display. The freshness guide method may include searching for a food identification code from an image captured by the camera, specifying a target food based on the food identification code, searching for at least one color code corresponding to the target food from the image, generating freshness information related to the target food based on the color code, and outputting the freshness information to the display.

According to one implementation, the searching for the color code may include searching for a partial region including the food identification code from an entire region of the image, determining a color code extraction region from the entire region of the image based on size and location of the partial region, and searching for the color code from the color code extraction region.

According to one implementation, the searching for the color code from the color code extraction region may include searching for at least one region having a color corresponding to the target food from the color code extraction region, wherein the color varies depending on the target food, and calculating an average color of the searched at least one region to determine the calculated average color as the color code.

According to one implementation, the color code may include a first color code for performing color conversion according to a first gas in the food accommodation space in which the target food is accommodated, and a second color code for performing a color conversion according to a second gas in the food accommodation space. A first color code extraction region corresponding to the first color code and a second color code extraction region corresponding to the second color code may be determined differently depending on the size and location of the partial region. The generating the freshness information may include generating first freshness information corresponding to the first color code based on an average color of the first color code extraction region, and generating second freshness information corresponding to the second color code based on an average color of the second color code extraction region.

According to one implementation, the freshness guide method may further include outputting the image to the display, and displaying a graphic object on the image to guide the color code extraction region.

According to one implementation, the generating the freshness information may include determining a freshness criterion of the target food, and generating freshness information related to the target food by applying the color code to the freshness criterion.

According to one implementation, the number of the color code searched for from the image may vary depending on the target food.

In addition, the packaging container may include a body having a food accommodation space defined by front, rear, both side (left and right) surfaces that extend upward from edges of a bottom surface, a cover covering an upper surface of the body to seal the food accommodation space, and having at least a portion transparent, a food identification code to allow food kept in the food accommodation space to be identified, and a gas sensor changing in color due to a gas in the food accommodation space.

According to one implementation, the packaging container may further include an adhesive member covering the food identification code and the gas sensor and adhered on a lower surface of the cover, and the adhesive member may be formed in a porous structure such that the gas in the food accommodation space is in contact with the gas sensor.

According to one implementation, the gas sensor may include a first gas sensor to perform a first color conversion in response to a first gas, and a second gas sensor to perform a second color conversion in response to a second gas different from the first gas.

### ADVANTAGEOUS EFFECTS

Hereinafter, effects of a freshness guide method and a freshness guide system according to the present disclosure will be described.

By an loT packaging system, a gas sensor may be disposed in a food accommodation space sealed together with food, and various information generated by the gas sensor may be transmitted to a server through a mobile terminal. By using big data stored in the server, freshness management and product management can be allowed in real time at each stage of food production, distribution, and consumption. This can lead to more efficient and safer food management.

In addition, a food consumer can directly check freshness of the sealed food by color conversion according to freshness or by tagging using a mobile terminal, and can determine whether to dispose food in a packaged state according to the checked freshness.

### BRIEF DESCRIPTION OF THE DRAWINGS

FIG. 1 is a block diagram of a mobile terminal in accordance with the present disclosure.
FIG. 2 is a block diagram illustrating a freshness guide system capable of guiding freshness of food sealed in a packaging container in accordance with the present disclosure.
FIG. 3 is a flowchart illustrating a freshness guide method capable of guiding freshness of food using the mobile terminal of FIG. 1
FIG. 4 is an exemplary view illustrating the operation of the mobile terminal according to the freshness guide method of FIG. 3.
FIGS. 5A and 5B are exemplary views illustrating the operation of the mobile terminal providing a visual guide line for detecting a color code.
FIGS. 6A and 6B are a perspective view and a cross-sectional view illustrating a packaging container by which freshness can be managed.
FIG. 7 is a perspective view illustrating a gas sensor in accordance with the present disclosure.

### BEST MODE FOR CARRYING OUT PREFERRED IMPLEMENTATIONS

Description will now be given in detail according to exemplary embodiments disclosed herein, with reference to the accompanying drawings. For the sake of brief description with reference to the drawings, the same or equivalent components may be provided with the same or similar reference numbers, and description thereof will not be repeated. In general, a suffix such as "module" and "unit" may be used to refer to elements or components. Use of such a suffix herein is merely intended to facilitate description of the specification, and the suffix itself is not intended to give any special meaning or function. In describing the present disclosure, if a detailed explanation for a related known function or construction is considered to unnecessarily divert the gist of the present disclosure, such explanation has been omitted but would be understood by those skilled in the art. The accompanying drawings are used to help easily understand the technical idea of the present disclosure and it should be understood that the idea of the present disclosure is not limited by the accompanying drawings. The idea of the present disclosure should be construed to extend to any alterations, equivalents and substitutes besides the accompanying drawings.

It will be understood that although the terms first, second, etc. may be used herein to describe various elements, these elements should not be limited by these terms. These terms are generally only used to distinguish one element from another.

It will be understood that when an element is referred to as being "connected with" another element, the element can be connected with the another element or intervening elements may also be present. In contrast, when an element is referred to as being "directly connected with" another element, there are no intervening elements present.

A singular representation may include a plural representation unless it represents a definitely different meaning from the context.

Terms such as "include" or "has" are used herein and should be understood that they are intended to indicate an existence of several components, functions or steps, disclosed in the specification, and it is also understood that greater or fewer components, functions, or steps may likewise be utilized.

Mobile terminals presented herein may be implemented using a variety of different types of terminals. Examples of such terminals include cellular phones, smart phones, user equipment, laptop computers, digital broadcast terminals, personal digital assistants (PDAs), portable multimedia players (PMPs), navigators, portable computers (PCs), slate PCs, tablet PCs, ultra books, wearable devices (for example, smart watches, smart glasses, head mounted displays (HMDs)), and the like.

By way of non-limiting example only, further description will be made with reference to particular types of mobile terminals. However, such teachings apply equally to other types of terminals, such as those types noted above. In addition, these teachings may also be applied to stationary terminals such as digital TV, desktop computers, and the like.

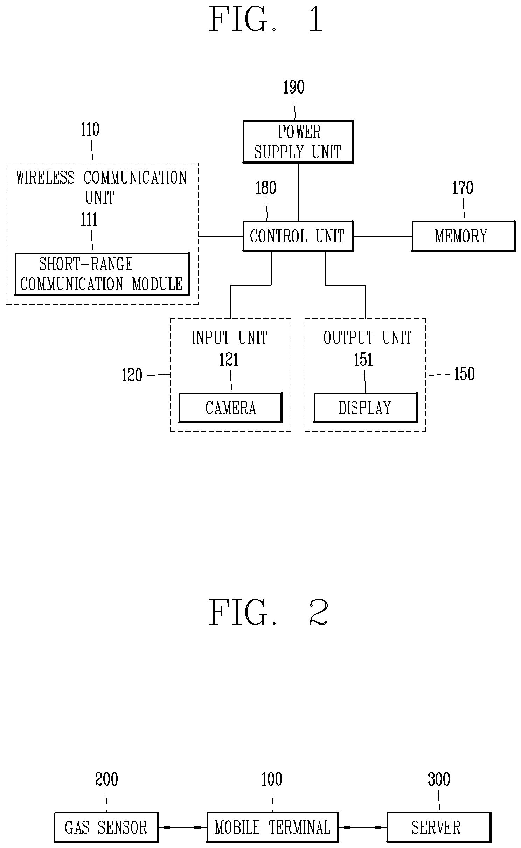

FIG. 1 is a block diagram of a mobile terminal in accordance with the present disclosure.

The mobile terminal 100 may be shown having components such as a wireless communication unit 110, an input unit 120, an output unit 150, a memory 170, a control unit 180, a power supply unit 190, and the like.

It is understood that implementing all of the illustrated components is not a requirement. Greater or fewer components may alternatively be implemented.

In more detail, the wireless communication unit 110 may typically include one or more modules which permit communications such as wireless communications between the mobile terminal 100 and a wireless communication system, communications between the mobile terminal 100 and another mobile terminal, or communications between the mobile terminal 100 and an external server. Further, the wireless communication unit 110 may typically include one or more modules which connect the mobile terminal 100 to one or more networks.

The short-range communication module 111 is configured to facilitate short-range communications. Suitable technologies for implementing such short-range communications include BLUETOOTHTM, Radio Frequency IDentification (RFID), Infrared Data Association (IrDA), Ultra-WideBand (UWB), ZigBee, Near Field Communication (NFC), Wireless-Fidelity (Wi-Fi), Wi-Fi Direct, Wireless USB (Wireless Universal Serial Bus), and the like. The short-range communication module 111 in general supports wireless communications between the mobile terminal 100 and a wireless communication system, communications between the mobile terminal 100 and another mobile terminal 100, or communications between the mobile terminal and a network where another mobile terminal 100 (or an external server) is located, via wireless area networks. One example of the wireless area networks is a wireless personal area network.

The input unit 120 may include a camera 121 or an image input unit for obtaining images or video, a microphone, which is one type of audio input device for inputting an audio signal, and a user input unit (for example, a touch key, a mechanical key, and the like) for allowing a user to input information. Data (for example, audio, video, image, and the like) may be obtained by the input unit 120 and may be analyzed and processed according to user commands.

The input unit 120 may be configured to input image information (or signal), audio information (or signal), data, or information input from a user. For inputting image information, the mobile terminal 100 may be provided with a plurality of cameras 121. Such cameras 121 may process image frames of still pictures or video obtained by image sensors in a video or image capture mode. The processed image frames can be displayed on the display 151 or stored in memory 170.

The output unit 150 may typically be configured to output various types of information, such as audio, video, tactile output, and the like. The output unit 150 may be shown having at least one of a display 151, an audio output module, a haptic module, and an optical output module. The display 151 may have an inter-layered structure or an integrated structure with a touch sensor in order to implement a touch screen. The touch screen may function as the user input unit which provides an input interface between the mobile terminal 100 and the user and simultaneously provide an output interface between the mobile terminal 100 and a user.

The display 151 may be generally configured to output information processed in the mobile terminal 100. For example, the display 151 may display execution screen information of an application program executing at the mobile terminal 100 or user interface (Ul) and graphic user interface (GUI) information in response to the execution screen information.

The display 151 may have an inter-layered structure or an integrated structure with a touch sensor in order to implement a touch screen. The touch screen may function as the user input unit 123 which provides an input interface between the mobile terminal 100 and the user and simultaneously provide an output interface between the mobile terminal 100 and a user.

The memory 170 may be typically implemented to store data to support various functions or features of the mobile terminal 100. For instance, the memory 170 may be configured to store application programs executed in the mobile terminal 100, data or instructions for operations of the mobile terminal 100, and the like. Some of these application programs may be downloaded from an external server via wireless communication. Other application programs may be installed within the mobile terminal 100 at time of manufacturing or shipping, which is typically the case for basic functions of the mobile terminal 100 (for example, receiving a call, placing a call, receiving a message, sending a message, and the like). Application programs may be stored in the memory 170, installed in the mobile terminal 100, and executed by the control unit 180 to perform an operation (or function) for the mobile terminal 100.

The control unit 180 typically functions to control an overall operation of the mobile terminal 100, in addition to the operations associated with the application programs. The control unit 180 may provide or process information or functions appropriate for a user by processing signals, data, information and the like, which are input or output by the aforementioned various components, or activating application programs stored in the memory 170.

Also, the control unit 180 may control at least some of the components illustrated in FIG. 1, to execute an application program that have been stored in the memory 170. In addition, the control unit 180 may control at least two of those components included in the mobile terminal 100 to activate the application program.

The power supply unit 190 may be configured to receive external power or provide internal power in order to supply appropriate power required for operating elements and components included in the mobile terminal 100. The power supply unit 190 may include a battery, and the battery may be configured to be embedded in the terminal body, or configured to be detachable from the terminal body.

At least part of the components may cooperatively operate to implement an operation, a control or a control method of a mobile terminal according to various embodiments disclosed herein. Also, the operation, the control or the control method of the mobile terminal may be implemented on the mobile terminal by an activation of at least one application program stored in the memory 170.

The mobile terminal 100 according to the present disclosure may include the components described above in FIG. 1, and may be used to guide freshness of food.

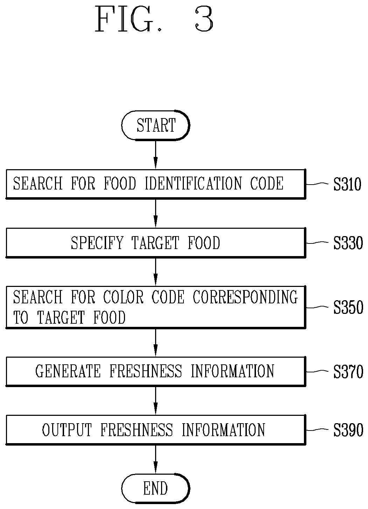

Hereinafter, a freshness guide system will be described with reference to FIG. 2, and a freshness guide method by the freshness guide system will be described with reference to FIG. 3.

FIG. 2 is a block diagram illustrating a freshness guide system capable of guiding freshness of food sealed in a packaging container in accordance with the present disclosure.

The freshness guide system may include a gas sensor 200, a mobile terminal 100, and a server 300.

The gas sensor 200 may be configured to sense a gas in a food accommodation space generated by a food stored in a packaging container.

The packaging container may have a food accommodation space in which food is accommodated or kept. The gas sensor 200 may be disposed in the food accommodation space to sense gas in the food accommodation space.

For example, the gas sensor 200 may generate gas information by sensing gas by power applied from the mobile terminal 100, in response to being tagged with the mobile terminal 100. The mobile terminal 100 may apply power to the gas sensor 200, in response to being tagged with the gas sensor 200. When power is applied, the gas sensor 200 may react with the gas in the food accommodation space so as to generate a difference in electrical conductivity.

In this case, the gas sensor 200 may include a substrate, an antenna and a signal processing module disposed on the substrate, an electrode pair disposed on the substrate and electrically connected to the signal processing module to receive power from the signal processing module, a conductive thin film disposed on the electrode pair, and a gas sensing film surrounding the conductive thin film and receiving power from the electrode pair so as to generate a difference in electrical conductivity according to a gas sensing reaction.

As another example, the gas sensor 200 may change in color so as to generate gas information, such as presence or absence of a detection target material (a material to be detected), concentration of the detection target material, etc. In other words, the gas sensor 200 may change in color due to the gas in the food accommodation space.

In this case, the gas sensor 200 may include a bioreceptor that selectively reacts with biomolecules as gases existing in the food accommodation space, and a color transducer that causes a change in color when such reaction occurs.

The color transducer may be made of a dye that expresses colors and changes in color according to various reactions such as a Lewis or Bronsted acid-base reaction, metal-based nanoparticles (gold, silver) caused due to localized surface plasmon resonance, vesicle, and the like.

For example, the gas sensor 200 may include a membrane composed of nanofibers having a core-shell structure. The core of the nanofiber may include a first polymer and the shell of the nanofiber may include a second polymer containing a dye material, which changes in color through reaction with gas molecules. The color-changing dye material may be bound to the shell of the nanofiber and an outer surface of the nanofiber.

The color-changing dye material may include a material that reacts with the gas molecules so as to exhibit color change characteristics due to the change in frequency of wavelength inside a visible ray region, the change in frequency of wavelength from inside to outside of the visible ray region, and the change in frequency or strength of wavelength from outside to inside of the visible ray region.

The color-changing dye material may include at least one of lead(II) acetate(Pb(CH3COO)2), iron(II) acetate(Fe(CH3COO)2), nickel(II) acetate(Ni(CH3COO)2), copper(II) acetate(Cu(CH3COO)2), cadmium acetate(Cd(CH3COO)2), cobalt(II) acetate(Co(CH3COO)2), manganese(II) acetate (Cu(CH3COO)2), bismuth(III) acetate(Co(CH3COO)3), silver(I) acetate(Ag(CH3COO)), silver nitride (AgNO3), o-Tolidine, m-Tolidine, bromophenol blue+TBAH, methyl red + TBAH, thymol blue + TBAH, fluorescein, bromocresol purple, bromophenol red, LiNO3, 5-10-15-20-tetraphenylporphyrinatozinc (II), ammonium molybdate (NH4)2MoO4, and 5-10-15-20-tetrakis(2,4,6-trimethylphenyl)porphyrinatozinc (II).

The color transducer of the gas sensor 200 may render various colors by using RGB (Red, Green, Blue) additive color mixing using three primary colors of light and CMYK (Cyan, Magenta, Yellow, Black) subtractive color mixing using three primary colors.

The color transducer may have a different color depending on concentration of a predetermined gas. Or, even for the same color, at least one of saturation and brightness may vary depending on concentration.

The first polymer and the second polymer may be polymers of at least one of polymethyl acrylate (PMA), polymethyl meta acrylate (PMMA), polyacrylic copolymer, polyvinyl acetate copolymer, polyvinyl acetate (PVAc), polyvinylpyrrolidone (PVP), polymethyl alcohol (PVA), polyfurfuryl alcohol (PPFA), polystyrene (PS), polystyrene copolymer, polyethylene oxide (PEO), polypropylene oxide (PPO), polyethylene oxide copolymer, polypropylene oxide copolymer, polycarbonate (PC), polyvinyl chloride (PVC), polycaprolactone, polyvinyl fluoride, polyvinylidene fluoride copolymer, polyimide, polyacrylonitrile (PAN), styrene-acrylonitrile (SAN), polyvinyl alcohol (PVA), polycarbonate (PC), polyaniline (PANI), polyvinyl chloride (PVC), poly(vinylidene fluoride)(PVDF), polyethylene terephthalate (PET), polypropylene (PP) and polyethylene (PE), styrene acrylonitrile (SAN).

The gas sensor 200 may sense one or more gases to generate gas information related to each gas. For example, the gas sensor 200 may include a first sensing module for performing a first color conversion in response to a first gas and a second sensing module for performing a second color conversion in response to a second gas different from the first gas.

When the plurality of sensing modules is provided in the gas sensor 200, they may be disposed to be spaced apart from each other by a predetermined interval. The plurality of sensing modules may be arranged in a matrix form so that the mobile terminal 100 can independently recognize the color conversion of each sensing module.

The gas sensor 200 may be configured to sense a different gas depending on a type of food sealed in the food accommodation space.

Meanwhile, the gas sensor 200 may include a food identification code configured to identify food sealed in the food accommodation space.

The food identification code may be made so that the mobile terminal 100 can specify food inside the food accommodation space using at least one of a camera and a short-range communication module. For example, the food identification code may be a barcode or QR code that can be sensed by the camera, or an RFID tag (or a transponder) that can be sensed by the short-range communication module.

The gas sensor 200 may further include a temperature sensor configured to change in color according to a temperature of the food accommodation space. The temperature sensor may have a first color in a first temperature range and a second color in a second temperature range. For example, the temperature sensor may have a blue color when detecting a temperature lower than a reference temperature and may have a red color when detecting a temperature higher than the reference temperature. At least one of saturation and brightness of color may vary according to a difference between the reference temperature and a current temperature. A user may visually check concentration of a specific gas in the food accommodation space and a temperature of the food accommodation space through the gas sensor 200.

The gas sensor 200 may be installed on a plurality of packaging containers in which different types of foods are packaged. The food identification code may be made to identify a manufacture date, a packaging data, a type, etc. related to food packed in the packaging container. For example, the first packaging container may be provided with a first gas sensor having a first food identification code for identifying a first food packed in the first packaging container, and the second packaging container may be provided with a second packaging container for identifying a second food packed in the second packaging container.

The gas sensor 200 may include a food identification code for identifying food sealed in the food accommodation space, and may generate gas information by sensing a gas changed by the food in the food accommodation space.

The mobile terminal 100 may receive various types of information from the gas sensor 200 disposed in the packaging container and transmit the received information to the server 200. The mobile terminal 100 may output freshness information related to a target food corresponding to the food identification code based on the gas information in at least one of visual, auditory, and tactile manners.

The mobile terminal 100 may search for the food identification code and the gas information using at least one of the camera and the short-range communication module, and output the searched food identification code and gas information through the wireless communication unit 110.

For example, the mobile terminal 100 may search for the food identification code by activating the camera, in response to a user input, and search for the gas information provided from the gas sensor 100. In this case, the gas information may be searched for from an image captured by the camera.

As another example, the mobile terminal 100 may search for the food identification code and the gas information, in response to being tagged with the gas sensor 200.

When receiving a plurality of different types of gas information from the gas sensor 200, the mobile terminal 100 may selectively use at least one type of gas information from among the plurality of gas information based on the food identification code.

For example, the gas sensor 200 may be massively produced by including a first gas module for generating first gas information by sensing a first gas, a second gas module for generating second gas information by sensing a second gas, and a third gas module for generating third gas information by sensing a third gas. When a first food identification code is received, the mobile terminal 100 may select only the first gas information to provide freshness information corresponding to the first gas information. When a second food identification code is received, the mobile terminal 100 may select the second and third gas information to provide freshness information corresponding to the second and third gas information. As such, for generating freshness information, all of the pieces of gas information received from the gas sensor 200 may not be used but different pieces of gas information may be selectively used depending on foods. Since there is no need to produce different types of gas sensors 200 optimized for each food, the gas sensor 200 can be massively produced.

Alternatively, the gas sensor 200 may be configured to sense different types of gases according to foods. For example, a first gas sensor disposed in a first food packaging container may be configured to sense a first gas, and a second gas sensor disposed in a second food packaging container may be configured to sense second and third gases.

The server 300 may determine a freshness criterion of the target food based on the food identification code and apply the color code to the freshness criterion to generate freshness information related to the target food.

For example, the server 300 may save in a memory a first freshness criterion for a first target food and a second freshness criterion for a second target food. When a food identification code received from the mobile terminal 100 corresponds to the first target food, freshness information may be generated according to the first freshness criterion. One the other hand, and when a food identification code corresponds to the second target food, freshness information may be generated according to the second freshness criterion. That is, since a different freshness criterion is applied to a target food even when the same color code is received, a completely different type of freshness information may be generated.

The server 300 may receive the food identification code and the gas information from the mobile terminal 100 and store them in the memory. The server 300 may specify foods based on food identification codes and manage gas information and/or freshness information corresponding to each food as big data.

Furthermore, the server 300 may find useful correlation in big data by applying an artificial intelligence (Al) technology to extract information to be executable in the future and use the extracted information for decision making. For example, inventory management and warehouse management can be performed using real-time freshness information, and waste generation can be minimized using actual freshness information related foods which are dynamically changing.

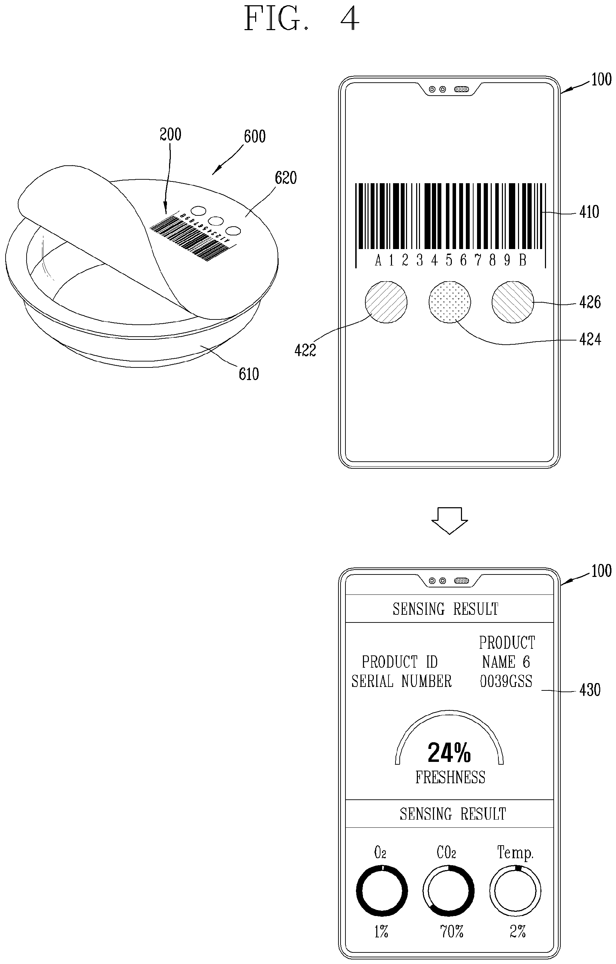

FIG. 3 is a flowchart illustrating a freshness guide method capable of guiding freshness of food using the mobile terminal of FIG. 1, and FIG. 4 is an exemplary view illustrating the operation of the mobile terminal according to the freshness guide method of FIG. 3.

The freshness guide method described in FIG. 3 may be executed through the mobile terminal 100 and/or the server 300.

First, the mobile terminal 100 may search for a food identification code (S310).

For example, the mobile terminal 100 may activate the camera 121 in response to a user input and output an image captured by the camera 121 to the display 151. The mobile terminal 100 may search for a food identification code from the image captured by the camera 121. For example, the food identification code may be a barcode or a QR code.

If the food identification code is not searched for, a warning such as "Food is not identified" may be output.

Next, the mobile terminal 100 may specify a target food based on the food identification code (S330).

The food identification code may include a food ID that can specify a food contained in a packaging container. The mobile terminal 100 may identify a name, a price, a type, a manufacture date, a packaging date, a manufacturing plant, and a distribution channel all related to the food, by using the food identification code through the server 300.

The food identification code may further include a real-time location system.

The mobile terminal 100 may specify the target food based on information stored in the memory, or may transmit the food identification code to the server 300 and specify the target food based on information received from the server 300.

Next, the mobile terminal 100 may search for one or more color codes corresponding to the target food from the image (S350).

The gas sensor 200 may sense one or more gases to generate gas information related to each gas. For example, the gas sensor 200 may include a first sensing module for performing a first color conversion in response to a first gas and a second sensing module for performing a second color conversion in response to a second gas different from the first gas.

When the plurality of sensing modules is provided in the gas sensor 200, the plurality of sensing modules may be disposed to be spaced apart from each other by a predetermined interval. The plurality of sensing modules may be arranged in a matrix form so that the mobile terminal 100 can independently recognize the color conversion of each sensing module.

Each sensing module may independently cause color conversion according to a gas to be detected. Accordingly, a color code generated by each sensing module may be searched for from the image.

The number of the color codes to be searched for from the image may vary depending on the target food. For example, it may be necessary to search for one color code for a first food and two color codes for a second food.

The mobile terminal 100 may transmit the identified food identification code to the server 300, and receive from the server 300 information related to at least one of the number and locations of color codes to be searched for from the image. Alternatively, the mobile terminal 100 may extract information related to at least one of the number and locations of color codes corresponding to the target food from the information stored in the memory.

The mobile terminal 100 may search for a partial region including the food identification code from an entire region of the image.

For example, as illustrated in FIG. 4, the mobile terminal 100 may search for a partial region 410, in which a captured barcode is present, from the entire region of the image output to the display 151.

The mobile terminal 100 may determine a color code extraction region from the entire region of the image based on size and location of the partial region. The number and locations of sensing modules provided in the gas sensor 200 may vary depending on the food identification code and the number and locations of the sensing modules may be preset.

The color codes may include a first color code for performing color conversion according to a first gas in the food accommodation space in which the target food is accommodated, and a second color code for performing color conversion according to a second gas in the food accommodation space. A first color code extraction region corresponding to the first color code and a second color code extraction region corresponding to the second color code may be determined differently depending on the size and location of the partial region.

For example, referring to FIG. 4, three color code extraction regions may be arranged below the partial region 410, in which a barcode is included, with being horizontally spaced apart from one another by predetermined intervals. That is, first to third color code extraction regions 422, 424, and 426 may be determined based on the partial region 410 extracted from the food identification code.

The arrangement of the food identification code and the color codes may be variously modified according to implementations, and the arrangement location of each color code may be identified based on the food identification code.

The mobile terminal 100 may extract each color code from each color code extraction region.

The mobile terminal 100 may search for at least one region having a color corresponding to the target food from the color code extraction region, and the color may vary depending on the target food. In addition, the mobile terminal 100 may calculate an average color of the searched at least one region and determine it as the color code.

For example, a color range in which a first color code can be expressed may be preset. A region outside the color range may correspond to meaningless data which is not related to the first color code. Accordingly, the mobile terminal 100 may search for at least one region, which is within a predetermined color range, from the color code extraction region. The mobile terminal 100 may calculate an average color of the searched at least one region and determine it as the color code. In the color code extraction region, a region out of the predetermined color range may be excluded when determining the color code. This may result in enhancing accuracy of color code extraction.

Next, the mobile terminal 100 may generate freshness information related to the target food based on the color code.

The mobile terminal 100 and/or the server 300 may generate freshness information related to the target food based on the color code.

Specifically, freshness information related to the target food may be generated by determining a freshness criterion of the target food and applying the color code to the freshness criterion. Since a different freshness criterion is applied depending on a target food, different freshness information may be generated even if the same color code is extracted. For example, even when the same nitrogen concentration is sensed in the food accommodation space, different freshness information may be generated depending on whether a food in the food accommodation space is a carrot or a Chinese cabbage.

Next, the mobile terminal 100 may output the freshness information in at least one of a visual, tactile, and auditory manners. For example, as illustrated in FIG. 4, freshness information 430 may be output to the display 151.

The mobile terminal 100 may generate first freshness information corresponding to the first color code based on an average color of the first color code extraction region, and generate second freshness information corresponding to the second color code based on an average color of the second color code extraction region.

The freshness information 430 may include a type and concentration of a gas determined by each color code, and a freshness value of food determined by all of gas information. The freshness information 430 may further include information related to a name, a manufacture date, a packaging date, a manufacturing plant, and the like of the food, which is specified by the food identification code.

Meanwhile, the mobile terminal 100 according to the present disclosure may help a user to easily capture a color code.

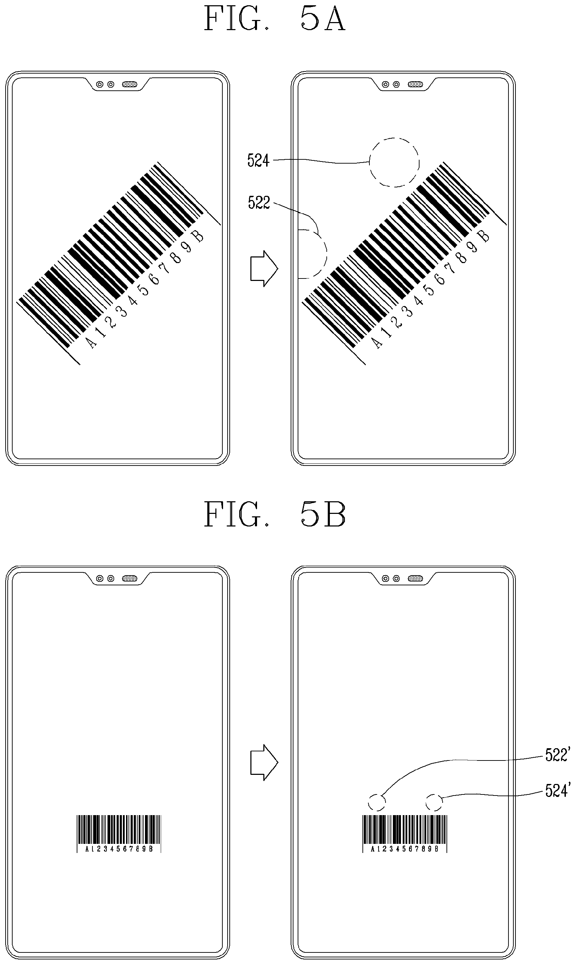

FIGS. 5A and 5B are exemplary views illustrating the operation of the mobile terminal providing a visual guide line for detecting a color code.

As described above with reference to FIG. 4, the number and locations of color codes to be searched for may be determined by the food identification code. A color code extraction region may vary depending on a partial region, which includes the food identification code, in the image captured by the camera. For example, as illustrated in FIGS. 5A and 5B, two color codes may be arranged above a food identification code to be spaced apart from each other in a horizontal direction.

The mobile terminal 100 may output the image captured by the camera to the display 151 and display a graphic object for guiding the color code extraction region on the image. Since the color code extraction region varies depending on the partial region including the food identification code, at least one of the number, location, and size of the graphic object may vary depending on the image.

For example, first and second graphic objects 522 and 524 corresponding to first and second color code extraction regions may be displayed as illustrated in FIG. 5A or displayed as illustrated in FIG. 5B according to a capturing method. The user may visually recognize which part corresponds to a color code based on graphic objects displayed on an image.

Meanwhile, the present disclosure provides a packaging container having the aforementioned gas sensor 200.

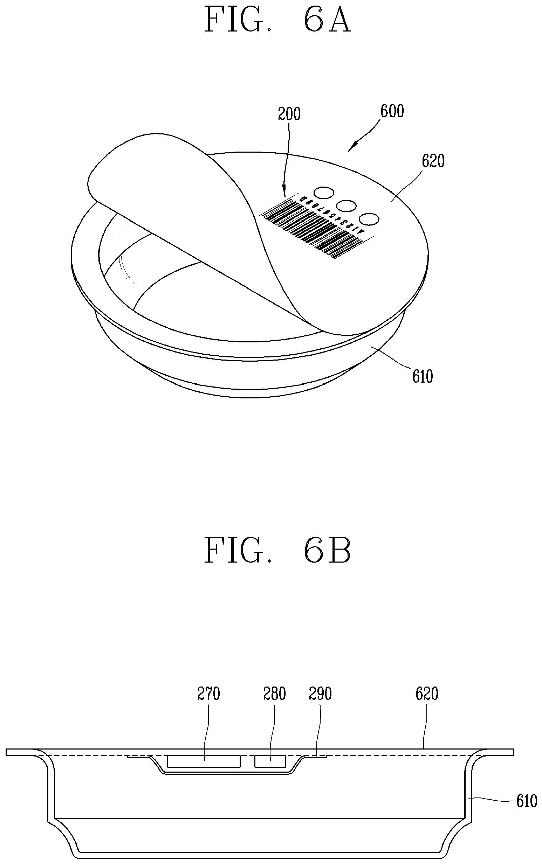

FIGS. 6A and 6B are a perspective view and a cross-sectional view illustrating a packaging container by which freshness can be managed.

A packaging container 600 may include a body 610 having a food accommodation space defined by front, rear, left and right surfaces which extend upward from edges of a bottom surface. In addition, the packaging container 600 may further include a cover 620 covering a top surface of the body 610 to seal the food accommodation space and having at least a portion made transparent.

The gas sensor 200 may be disposed on one surface of the cover 620 facing the food accommodation space.

The gas sensor 200 may include a food identification code 270 for identifying food kept in the food accommodation space, and at least one sensing module 280 that changes in color due to a gas in the food accommodation space.

The packaging container 600 may further include an adhesive member 290 that covers the food identification code 270 and the sensing module 280 and is adhered on a lower surface of the cover 620.

The adhesive member 290 may be formed in a porous structure so that the gas in the food accommodation space is in contact with the gas sensor.

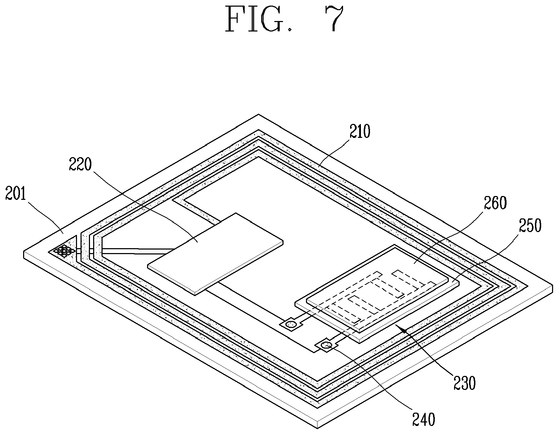

FIG. 7 is a perspective view illustrating a gas sensor in accordance with the present disclosure.

An application for providing freshness information regarding food may be installed in the mobile terminal 100. When the mobile terminal 100 and the gas sensor 200 are located in a short distance from each other, they may be connected to each other through short-range communication. For example, RFID or NFC may be used as such short-range communication.

The mobile terminal 100 may transmit a wireless signal to the gas sensor 200 through short-range communication. The gas sensor 200 may then be operated using a current induced by the wireless signal so as to measure gas concentration.

When the gas sensor 200 measures the gas concentration and transmits a gas sensing signal corresponding to the measured gas concentration to the mobile terminal 100, the mobile terminal 100 may display a user interface screen on the display 151 so as to provide freshness information to the user through the application.

Referring to FIG. 7, in a gas sensing tag, an antenna 210 and a signal processing module 220 may be disposed on a substrate 201 and a sensor module 230 may be provided to be electrically connected to the signal processing module 220.

The sensor module 230 may include an electrode pair 240, a conductive thin film 250, and a gas sensing film 260.

The substrate 201 may be an insulating substrate such as a synthetic resin film, glass, ceramic, or silicon substrate. The antenna 210 may be patterned on a periphery of the substrate 201 and the signal processing module 220 may be mounted to be electrically connected to the antenna pattern 210.

The electrode pair 240 may be disposed on the substrate 201 and electrically connected to the signal processing module 220 so as to receive power from the signal processing module 220.

Specifically, the electrode pair 240 may be connected to a rectifier circuit 24 for supplying DC power and a control unit 26 which is a measurement part that receives and processes a potential difference signal.

The electrode pair 240 may be, for example, made of a metal selected from a group including gold (Au), platinum (Pt), silver (Ag), nickel (Ni), copper (Cu), tungsten (W), aluminum (Al), or its alloy.

The conductive thin film 250 may be disposed on the electrode pair 240. The conductive thin film 250 may facilitate decomposition reaction and combination reaction of a gas to be sensed so as to increase response speed and sensitivity of the gas sensor. That is, the conductive thin film 250 may act as a catalyst to promote a sensing reaction of the gas sensing film 260 with respect to the gas to be sensed.

The gas sensing film 260 may be disposed on the conductive thin film 250 to surround the conductive thin film 250. The gas sensing film 260 may receive power from the electrode pair 240 to generate a difference in electrical conductivity according to the sensing reaction of the gas.

The gas sensing film 260 may be at least one selected from a group of semiconductor metal oxides including titanium oxide (TiO4), tin oxide (SnO2), or an alloy thereof. However, the present disclosure is not limited thereto, and various types of materials in which electrical conductivity varies may be used as the gas sensing film 5.

For example, when a reduction gas, which is a gas to be sensed, is exposed using tin oxide as the gas sensing film 260, electrical conductivity of the tin oxide may be restored by a surface reaction of the tin oxide. The reduction gas may remove oxygen adsorbed on the tin oxide. At this time, conduction electrons captured by the oxygen may move into the tin oxide, thereby increasing the electrical conductivity of the tin oxide.

As described above, the gas sensing film 260 may generate a difference between electrical conductivity when the gas is not exposed and electrical conductivity when the gas is exposed, and a potential difference may be generated in the electrode pair 240 due to the difference in electrical conductivity of the gas sensing film. The gas sensor 30 may sense the gas to be sensed by using the potential difference.

The present disclosure can be implemented as computer-readable codes (applications or software) in a program-recorded medium. The method of controlling the autonomous vehicle can be realized by a code stored in a memory or the like.

The computer-readable medium may include all types of recording devices each storing data readable by a computer system. Examples of such computer-readable media may include hard disk drive (HDD), solid state disk (SSD), silicon disk drive (SDD), ROM, RAM, CD-ROM, magnetic tape, floppy disk, optical data storage element and the like. Also, the computer-readable medium may also be implemented as a format of carrier wave (e.g., transmission via an Internet). The computer may include the processor or the control unit. Therefore, it should also be understood that the above-described embodiments are not limited by any of the details of the foregoing description, unless otherwise specified, but rather should be construed broadly within its scope as defined in the appended claims, Therefore, all changes and modifications that fall within the metes and bounds of the claims, or equivalents of such metes and bounds are therefore intended to be embraced by the appended claims.

## Claims

1. A freshness monitoring system comprising:
a gas sensor (200) having a food identification code for identifying food sealed in a food accommodation space, and to generate gas information by sensing a gas changed by the food in the food accommodation space;
a mobile terminal (100) to search for the food identification code and the gas information using at least one of a camera and a short-range communication module, and to output the food identification code and the gas information through a wireless communication unit; and
a server (300) to receive the food identification code and the gas information from the mobile terminal 100 and store the same in a memory, and
wherein the mobile terminal (100) outputs the freshness information related to a target food corresponding to the food identification code based on the gas information in at least one of visual, auditory and tactile manners.

2. The system of claim 1, wherein the gas sensor (200) changes in color due to the gas in the food accommodation space.

3. The system of claim 2, wherein the mobile terminal (100) searches for the food identification code from an image captured by the camera, searches for at least one color code corresponding to the target food from the image, and
wherein the freshness information related to the target food is determined by the color code.

4. The system of claim 3, wherein the mobile terminal (100) searches for a partial region including the food identification code from an entire region of the image, determines a color code extraction region from the entire region of the image based on size and location of the partial region, and searches for the color code from the color code extraction region.

5. The system of claim 4, wherein the mobile terminal (100) searches for at least one region having a color corresponding to the target food from the color code extraction region, and calculates an average color of the searched at least one region, and determines the calculated average cover as the color code, and
wherein the color changes depending on the target food.

6. The system of claim 4, wherein the mobile terminal (100) displays the image on a display, and outputs a graphic object on the image to guide the color code extraction region.

7. The system of claim 3, wherein the server (300) determines a freshness criterion of the target food based on the food identification code, and generates the freshness information related to the target food by applying the color code to the freshness criterion.

8. The system of claim 3, wherein the number of the color code searched for from the image varies depending on the target food.

9. The system of claim 1, wherein the mobile terminal (100) applies power to the gas sensor (200), in response to being tagged with the gas sensor (200), and
wherein the gas sensor (200) generates a difference in electrical conductivity through reaction with the gas in the food accommodation space when the power is applied.

10. The system of claim 9, wherein the gas sensor (200) comprises:
a substrate;
an antenna and a signal processing module disposed on the substrate;
an electrode pair disposed on the substrate and electrically connected to the signal processing module to receive power from the signal processing module;
a conductive thin film disposed on the electrode pair; and
a gas sensing film disposed to surround the conductive thin film and to generate a difference in electrical conductivity through gas sensing reaction by receiving power from the electrode pair.

11. A freshness guide method for guiding freshness of food using a mobile terminal having a camera and a display, the method comprising:
searching for a food identification code from an image captured by the camera;
specifying a target food based on the food identification code;
searching for at least one color code corresponding to the target food from the image;
generating freshness information related to the target food based on the color code; and
outputting the freshness information to the display.

12. The method of claim 11, wherein the searching for the color code comprises:
searching for a partial region including the food identification code from an entire region of the image;
determining a color code extraction region from the entire region of the image based on size and location of the partial region; and
searching for the color code from the color code extraction region.

13. The method of claim 12, wherein the searching for the color code from the color code extraction region comprises:
searching for at least one region having a color corresponding to the target food from the color code extraction region, the color varying depending on the target food; and
calculating an average color of the searched at least one region to determine the calculated average color as the color code.

14. The method of claim 13, wherein the color code includes a first color code for performing color conversion according to a first gas in the food accommodation space in which the target food is accommodated, and a second color code for performing a color conversion according to a second gas in the food accommodation space,
wherein a first color code extraction region corresponding to the first color code and a second color code extraction region corresponding to the second color code are determined differently depending on the size and location of the partial region, and
wherein the generating the freshness information comprises:
generating first freshness information corresponding to the first color code based on an average color of the first color code extraction region; and
generating second freshness information corresponding to the second color code based on an average color of the second color code extraction region.

15. The method of claim 12, further comprising:
outputting the image to the display; and
displaying a graphic object on the image to guide the color code extraction region.

16. The method of claim 11, wherein the generating the freshness information comprises:
determining a freshness criterion of the target food; and
generating freshness information related to the target food by applying the color code to the freshness criterion.

17. The method of claim 11, wherein the number of the color code searched for from the image varies depending on the target food.

18. A packaging container comprising:
a body having a food accommodation space defined by front, rear, left and right surfaces that extend upward from edges of a bottom surface;
a cover covering an upper surface of the body to seal the food accommodation space, and having at least a portion transparent;
a food identification code to allow food kept in the food accommodation space to be identified; and
a gas sensor changing in color due to a gas in the food accommodation space.

19. The container of claim 18, further comprising an adhesive member covering the food identification code and the gas sensor and adhered on a lower surface of the cover,
wherein the adhesive member is formed in a porous structure such that the gas in the food accommodation space is in contact with the gas sensor.

20. The container of claim 18, wherein the gas sensor comprises:
a first gas sensor to perform a first color conversion in response to a first gas; and
a second gas sensor to perform a second color conversion in response to a second gas different from the first gas.
